(19) **Europäisches Patentamt**

**European Patent Office**

**Office européen des brevets**

(11) **EP 1 421 924 A2**

(12) **EUROPEAN PATENT APPLICATION**

(43) Date of publication:
26.05.2004 Bulletin 2004/22

(51) Int Cl.7: **A61F 13/02**, A61F 13/00

(21) Application number: 03256782.8

(22) Date of filing: 28.10.2003

| | |
|---|---|
| (84) Designated Contracting States: **AT BE BG CH CY CZ DE DK EE ES FI FR GB GR HU IE IT LI LU MC NL PT RO SE SI SK TR** Designated Extension States: **AL LT LV MK** | (72) Inventors: <br> • **Haddock, Teresa H.** **Hudson Ohio 44236 (US)** <br> • **Martin, Alison Brooke** **Lawrenceville New Jersey 08648 (US)** <br> • **Jones, Kelly C.** **Bensalem Pennsylvania 19020 (US)** |
| (30) Priority: **31.10.2002 US 285127** | |
| (71) Applicant: **JOHNSON & JOHNSON CONSUMER COMPANIES, INC.** **Skillman, NJ 08558 (US)** | (74) Representative: **Mercer, Christopher Paul et al** **Carpmaels & Ransford** **43, Bloomsbury Square** **London WC1A 2RA (GB)** |

(54) **Textured breathable films and their use as backing material for bandages**

(57)     A breathable film for contact with skin wherein the film has texture variations is disclosed. The film is useful as a backing material for an adhesive bandage, and a bandage employing the film is easily applied to the skin of a user without inadvertently sticking to itself or surfaces other than the skin.

FIG. 6

EP 1 421 924 A2

**Description**

Background Of The Invention

This patent relates to textured breathable films and the use of textured breathable films as backing materials for bandages. The textured breathable films are easy to handle and bandages made from them are readily applied to the skin of a user.

It is well known to apply adhesive bandages (also known as wound dressings) to a wound to protect the wound and keep the wound clean. Most commonly, adhesive bandages are made from a polyethylene or polyvinyl chloride backing material. One side of the backing material includes a wound-contacting pad, which functions to keep the wound clean and to cushion the wound. An adhesive is applied to the backing material alongside the edges or around the perimeter of the wound-contacting pad to hold the bandage in place over the wound.

A polyethylene backing material does not allow water vapor to leave the surface of the skin covered by the bandage. This leads to discomfort for the user. This is also true of backing materials made from polyvinyl chloride. In order to overcome this problem, backing materials made from polyethylene, polyvinyl chloride and the like materials are typically perforated to allow water vapor to leave the surface of the skin.

Although perforated or apertured films are useful as backing materials, there is a concern that the perforations or apertures may allow liquid water to reach the surface of the skin and/or the wound underlying the bandage. The presence of water may promote bacterial growth which, in turn, might lead to infection of the wound.

Therefore, there is a need for a bandage that allows water vapor to evaporate from the surface of the skin under the bandage (i.e., is "breathable"), but does not allow liquid water to reach the surface of the skin under the bandage (i.e., is "waterproof").

The use of thin breathable films, e.g. a polyurethane film having a thickness in the range of 0.025 mm, as a backing material for a wound dressing has been practiced since the 1970s. United States Patent No. 3,645,835 discloses this type of adhesive dressing for blocking bacteria and liquid water from reaching the wound, but allowing oxygen to penetrate the dressing from the atmosphere and allowing moisture from the skin of the patient to escape from beneath the dressing.

The moisture vapor transmission rate ("MVTR") indicates the degree of breathability of a film, the higher the MVTR, the higher the degree of breathability. In order to obtain the desired MVTR, these types of films are generally thin, i.e., less than 0.05 mm in thickness. Because of the nature of the polymers used for breathable films, breathable films made at a thickness of about 0.05 mm or less are usually flexible, limp, flimsy and hard to handle. When adhesive is applied to the film to enable the film to adhere to the skin, the film tends to stick to itself wherever adhesive surfaces touch each other. This makes it difficult to apply thin breathable film dressings to the skin.

To overcome this problem, delivery systems have been designed to handle these types of dressings. United States Patent Nos. 4,413,621 and 4,485,809 are two examples. One disadvantage of thin breathable film dressings with delivery systems is that it is sometimes difficult for users to figure out how to use them. Another disadvantage of thin breathable film dressings with delivery systems is that it is sometimes difficult for users to actually use the delivery system.

Another method to improve the ability to handle thin breathable films is taught in United States Patent No. 4,846,164. The '164 patent teaches the use of composites for backing materials. These backing materials combine thin films with other materials. United States Patent No. 4,773,409 also teaches the use of composites for backing materials for bandages. The composites include a polyurethane film and a polyurethane foam containing water dispersible or water swellable agents. Bandages using such composite backing materials are generally more expensive to produce than bandages using thin films as backing materials. Additionally, the presence of water swellable agents might not be desirable, as the result would be a moist surface against the wound, which might promote undesirable bacterial growth.

There are commercially available bandages that have embossed non-breathable backings. Examples of such bandages include TARGET Brand Water Resistant Bandages, Wegmans® Waterproof Bandages, and CVS® WaterShield Bandages.

Despite the disclosure of the references, there is a continuing need for a bandage that is breathable, waterproof, and easy to handle and apply to a wound.

Summary Of The Invention

The present invention provides a breathable and waterproof film for contact with skin wherein said film includes texture variations. The present invention further provides an adhesive bandage in which the backing material comprises a breathable film backing having texture variations, the breathable film having a first side and a second side, an adhesive coating on at least one of said sides, and a wound-contacting pad applied to a portion of the adhesive on said at least one side.

Brief Description of the Drawings

The present invention will be better understood with reference to the accompanying drawings in which:

Fig. 1 is a fragmentary perspective view of one embodiment of a textured film in accordance with the present invention;

Fig 2 is a schematic representation of an embossing apparatus which can be used to make textured films in accordance with the present invention;

Fig. 3 is a greatly enlarged fragmentary view, taken in the direction of view line 3-3 in Fig. 2, of an embossing roller having a plurality of hexagonal bosses extending from its outer surface, said embossing roller being useful for making textured films in accordance with the present invention;

Fig. 4 is a cross-section taken along line 4-4 of Fig. 3;

Fig. 5 is a top plan view of the textured film shown in Fig. 1; and

Fig. 6 is a cross-section taken along line 6-6 of Fig. 5.

Detailed Description Of The Invention

The present invention provides a film that is breathable and monolithic. As used herein, the term " breathable" means that the film has an MVTR of at least 500 $g/m^2$/24 hrs, preferably above 1,000 $g/m^2$/24 hrs, and more preferably above 2,000 $g/m^2$/24 hrs. As used herein, the term "monolithic" means that the films do not have micropores, perforations, or apertures. The thickness of the film is not critical, but typically ranges from about 0.01 mm to about 0.075 mm, preferably from about 0.025 mm to about 0.050 mm. Suitable breathable films include, but are not limited to, polyurethane films, polyurethane foams, polyolefin films, polyester films, polyvinyl chloride films, silicone films, and polyetheramide films.

The breathable film is textured by means known in the art. As used herein, the term "textured" refers to the presence on one major surface of the film of raised portions that are not perforated or apertured. One means of providing texture to a film is to emboss it. Generally, the film to be embossed is passed between the nip of two rollers under pressure. Optionally, one or both of the rolls may be heated. One roller (the "back-up" roller) has a smooth, resilient surface and the other roller (the "embossing" roller) has protrusions, or bosses, in the shape of the raised portions desired in the film. The film resulting from such an embossing process comprises a male side 27 and a female side 28. The male side has raised protrusions in the form of the bosses on the embossing roller. The female side has depressions corresponding to the configuration of the bosses on the embossing roller.

Another means of providing texture to a film is to pass the film over a roller having holes in the shape of the texture variations. Sufficient vacuum is applied through the roller to pull the film into the holes and create the desired texture variations without rupturing the film.
A third means of providing texture variations to a film is to extrude a molten, film-forming material (e.g., a polyurethane resin) onto a surface having texture variations and cool the material to form the textured film. Other means of providing texture variations to films are known in the art. The embossing process is preferred for use in the practice of the present invention.
The texture variations provide the film with a degree of rigidity which makes it easier to apply the film to a wound without the film inadvertently sticking to itself or surfaces other than the skin. The shape of the texture variations is not critical. Suitable shapes of texture variations include, but are not limited to, circles, hemispheres, hearts, moons, stars, ellipses, hills and valleys, triangles, squares, numbers, and alphabetical letters. The texture variation may take an annular form, if desired. The number of texture variations may range from about 10 per $cm^2$ to about 300 per $cm^2$, preferably from about 25 per $cm^2$ to about 150 per $cm^2$. The height of the texture variations may range from about 0.01 mm to about 1 mm, preferably from about 0.1 mm to about 0.4 mm.
Films according to the present invention have a first side and a second side. At least one side of the film has an adhesive applied thereto. The adhesive functions to adhere the film to the skin during use. In the case of an adhesive bandage, the adhesive also functions to secure a wound-contacting pad to the film. The adhesives may be hot melt adhesives. Examples of suitable adhesives include, but are not limited to those based on styrenic block copolymers and tackifying resins such as HL-1491 from HB-Fuller Co. (St. Paul MN), H-2543 from ATO-Findley (Wawatausa, WI), and 34-5534 from National Starch & Chemical (Bridgewater, NJ). Ethylene copolymers including ethylene vinyl acetate may also be useful.
Suitable adhesives also include acrylic based, dextrin based, and urethane based adhesives as well as natural and synthetic elastomers. The adhesives may also include amorphous polyolefins including amorphous polypropylene, such as HL-1308 from HB Fuller or Rextac RT 2373 from Huntsman (Odesssa, TX). The adhesive may comprise Kraton® Brand synthetic rubber and the like, or natural rubber compounded, if desired, with tackifiers, antioxidants and processing aids.
The adhesive can be applied in the molten stage, sprayed, or slot die coated. The spray can be applied by control coating, control weaving, control fiberization, meltblowing, flexo coating, screen printing, or other coating methods. The adhesive coating may be continuous or discontinuous as is known in the art. The amount of adhesive typically applied is well known in the art. Generally, the adhesive coating weight may vary from about 20 grams per square meter ("gsm") to about 100 gsm. Other coating weights may be used if desired.

As is common practice, the adhesive surface may be protected prior to use by one or more pieces of release paper. Suitable release papers are well known in the art.

For use in bandages, the adhesive coated, breathable film having texture variations has a wound-contacting pad secured thereto. The pad keeps the wound clean and dry, absorbs exudate from the wound, and provides cushioning for the wound. As is known in the art, the wound-contacting pad may be completely surrounded by the adhesive, resulting in a so-called "island pad" configuration. Alternatively, the wound-contacting pad may be co-extensive in width with the backing material but shorter in length, resulting in a so-called "strip" bandage.

The wound-contacting pad may comprise any absorbent material. Suitable absorbent materials include absorbent gels; hydrocolloids; alginate fibers; rayon fibers; natural fibers, such as, but not limited to, cotton and wood pulp fibers; and synthetic fibers, such as, but not limited to, polyester fibers, polyamide fibers, and polyolefm fibers, copolymers thereof, and mixtures thereof. The fibers may be bicomponent fibers. For example, the fibers may have a core of one polymer, and a sheath of a different polymer.

Physical integrity of the wound-contacting pad may be achieved by the frictional engagement of the fibers or blend of fibers of which it is constituted. Physical integrity of the wound-contacting pad may be enhanced, if necessary or desired, by the application thereto of a binder material as is known in the art. Alternatively, heat sensitive fibers having a relatively low melting point can be included in the wound-contacting pad, which can then be heat-treated, e.g., with hot air, to tackify the heat sensitive fibers and thereby provide the desired physical integrity.

Although not a necessary feature of the present invention, it is preferable to cover the upper surface of the wound-contacting pad with a wound release means. As is known in the art, an apertured plastic film or netting, e.g., one made from polyethylene or the like, may serve as such wound release means. Apertured plastic films suitable for covering the wound-contacting pad are commercially available, e.g., from Applied Extrusion Technology, Middletown, Delaware 19709 USA.

The adhesive coated film of the present invention has an MVTR above 500 g/m$^2$/24 hrs, preferably above 1,000 g/m$^2$/24 hrs, even more preferably, above 2000 g/m$^2$/24 hrs when tested as set forth hereinafter. Since the film is breathable, there is no need for perforation as is commonly used with otherwise non-breathable backing materials made from polyethylene, polyvinyl chloride and the like. The bandage has a bulk thickness of at least 0.035 mm.

Bandages are typically tested on an Instron testing machine. The bandages of this invention have a recovery of at least about 70% when elongated to 50% of their original length and recovery of at least about 50% when elongated to 20% of their original length.

Preferably, the recovery is over about 90% at both elongations. At recovery levels in this range the bandage will conform to body movements when used on joints such as the knuckles.

Stretch and Recovery Test

Samples to be tested were first conditioned at 50% relative humidity and 75°F for at least 4 hours. An Instron testing machine was utilized with the pulling clamp speed set at 12.5 cm per minute. The distance (i. e., gage length) between the sample clamps was 10 cm at the start of the test. The machine had a cycling control that was adjustable to permit the stressing of the sample to a selected distance and returning it immediately to the selected gage length at the same speed. The faces on the jaws of the machine measured at least 2.5 cm x 3.75 cm, with the long dimension perpendicular to the direction of application of the load. The jaws had smooth gripping surfaces.

The samples were clamped firmly and squarely in the jaws of the clamps. The cycle extension limits were set to reflect the amount of extension or elongation desired (e.g., 20% extension required 20% x 10 cm gauge length = 2 cm maximum limit on the cycle control). Force was applied to the test samples at a rate of 12.5 cm per minute, and the sample was stressed to the desired elongation. The sample was then returned at the same speed to the test cycle starting point. The % recovery was calculated according to the following formula:

$$\% \, re\,\mathrm{cov}\,ery = \left( \frac{le - l_t}{le - lo} \right) x \, 100$$

where lo = original length of sample, le = fully extended length of sample, $l_t$ = length of sample when fully relaxed.

Moisture Vapor Transmission Rate

The MVTR was measured following ASTM method F1249. The MVTR was considered acceptable if it was greater than 500 g/m$^2$/24hrs.

The following examples are provided for illustrative purposes. The invention should not be construed as being limited to the details thereof.

Example 1 - Adhesive Coated Breathable Film

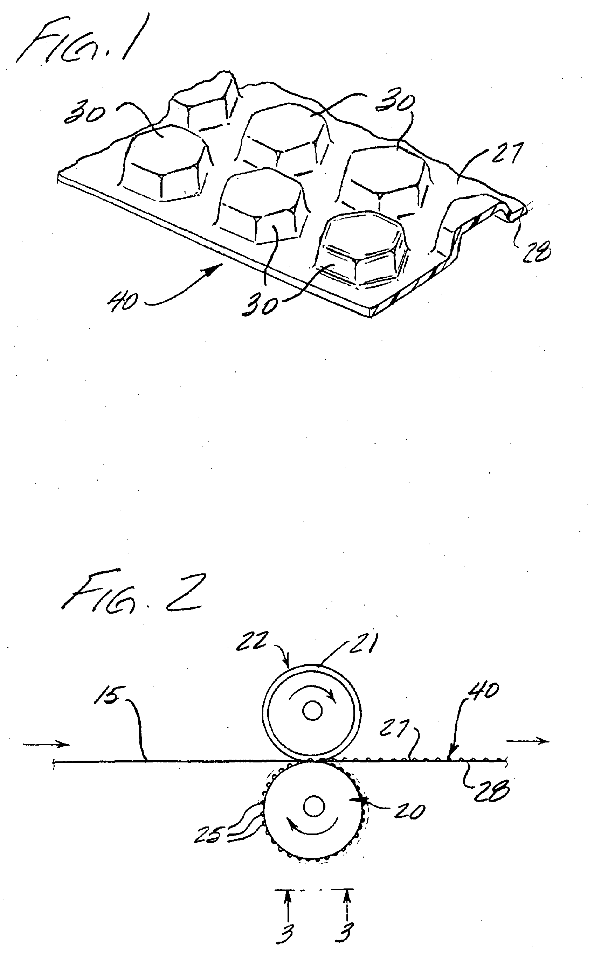

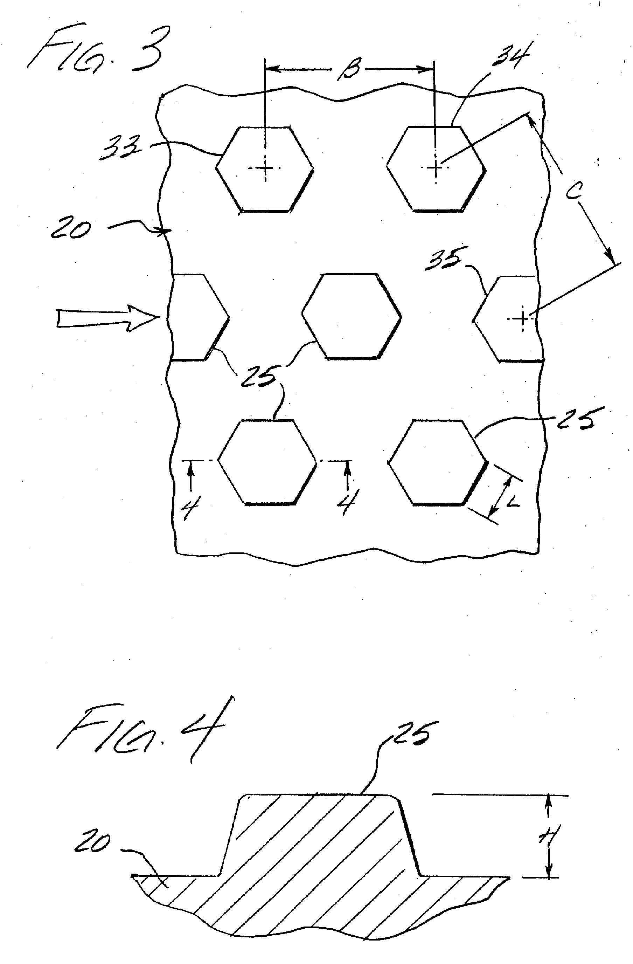

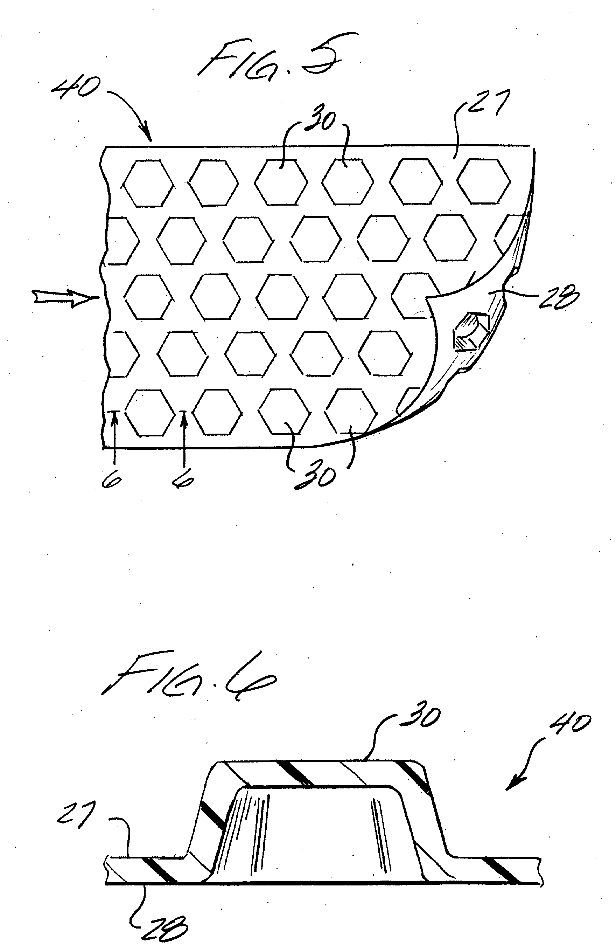

A 0.025 mm thick opaque polyester-based starting film 15 (HYTREL 4778 from DuPont Chemical Company) was embossed by passing the film through the nip of a resilient backup roller 22 and an embossing roller 20. The backup roller was rubber coated and the rubber coating 21 had a smooth surface. The embossing roller, indicated by numeral 20 in Fig. 3, had a plurality of bosses 25 extending outwardly from the surface thereof. The bosses 25 were hexagonal in configuration, with each side of the hexagon having a length, L, of about 0.8 mm. Bosses 25 were arranged in a staggered pattern as shown in Fig. 3. There were about 77 bosses/$cm^2$ of embossing roller surface area. The center-to-center distance, B, between adjacent bosses 33, 34 in a row parallel to the longitudinal axis (indicated by the directional arrow in Fig. 3) of the embossing roller was about 1.4 mm. The center-to-center distance, C, between a given boss 34 in one axially extending row and a diagonally opposite boss 35 in the next adjacent axially extending row was about 1.4 mm. The hexagonal bosses had a height, H, of about 0.25 mm.

The resulting film 40 had a thickness of about 0.25 mm in its embossed regions. The unembossed regions had a thickness which was about the same as that of the unembossed starting film 15, i.e., about 0.025 mm. There were 77 embossments/$cm^2$ of embossed film. The embossments 30 on the resulting film had L and H dimensions and center-to-center spacings generally equivalent to the aforementioned L and H dimensions and center-to-center spacings of bosses 25 on embossing roller 20. The embossed film had an MVTR of 3,053 g/$m^2$/24 hrs. The film was elongated 50% on the INSTRON testing machine and found to have a recovery of 84%. A silicone-based adhesive was applied at 50 grams per square meter on a release paper. After drying, the adhesive on the adhesive coated release paper was transfer-coated to the female (unembossed) side of the embossed film. The resulting adhesive coated embossed film had an MVTR of 800 g/$m^2$/24 hrs.

### Example 2 - Breathable Bandage

Bandages were prepared by cutting 7.5 cm long, 2.54 cm wide backing strips from the adhesive-coated breathable film of Example 1. Wound-contacting pads (2.2 cm x 1.2 cm, 3.7 ounce/$yd^2$, 90/10 polyester/rayon) were placed on the adhesive coated side of the backings. The wound-contacting pads were centered end-to-end and side-to-side of the backing. The resulting bandages were not flimsy and were easily applied to the skin.

### Example 3 - Changing The Orientation Of The Backing

Examples 1 and 2 were repeated except the adhesive was applied to the male (embossed) surface of the breathable film. The bandages were not flimsy and were easily applied to the skin.

### Example 4 - Changing The Adhesive

Examples 1 and 2 were repeated except a transparent polyester-based film (HYTREL from DuPont Chemical Company) was utilized and a poly(2-ethylhexyl acrylate) adhesive was substituted for the silicone based adhesive. The bandages were not flimsy and were easily applied to the skin.

### Example 5 - Waterproof Test

The bandages prepared above were tested to determine whether they were waterproof. Panelists applied the bandages to their fingers, then immersed their hands in 40°C water for ten minutes. The area of skin under the bandages remained dry after ten minutes soaking in water. Therefore, the bandages of the present invention are waterproof.

### Claims

1. A breathable film for contact with skin wherein the film comprises texture variations.

2. A film according to claim 1 wherein the texture variations are embossments.

3. A film according to claim 2 comprising from about 10 embossments per $cm^2$ to about 300 embossments per $cm^2$.

4. A film according to claim 2 comprising ranges from about 25 embossments per $cm^2$ to about 150 embossments per $cm^2$.

5. A film according to claim 1 wherein the depth of the texture variations ranges from about 0.01 mm to about 1 mm

6. A film according to claim 1 wherein the depth of the texture variations ranges from about 0.1 mm to about 0.4 mm.

7. A film according to claim 1 wherein the texture variations are in a shape selected from the group consisting of circles, hemispheres, hearts, moons, stars, ellipses, hills and valleys, triangles, squares, numbers, and alphabetical letters.

8. A film according to claim 1 wherein the texture variations are in the form of hemispheres.

9. A bandage comprising:

   a breathable film backing having texture variations, said breathable film having a first side and a second side;

   an adhesive coating on at least one of said first and second sides of said breathable film; and

   a wound-contacting pad applied to a portion of said adhesive coating.

10. A bandage according to claim 9 wherein the texture variations are embossments.

11. A bandage according to claim 10 wherein the film comprises from about 10 embossments per cm$^2$ to about 300 embossments per cm$^2$.

12. A bandage according to claim 10 wherein the film comprises from about 25 embossments per cm$^2$ to about 150 embossments per cm$^2$.

13. A bandage according to claim 9 wherein the depth of the texture variations ranges from about 0.01 mm to about 1 mm.

14. A bandage according to claim 9 wherein the depth of the texture variations ranges from about 0.1 mm to about 0.4 mm.

15. A bandage according to claim 9 wherein the texture variations are in a shape selected from the group consisting of circles, hemispheres, hearts, moons, stars, ellipses, hills and valleys, triangles, squares, numbers, and alphabetical letters.

16. A bandage according to claim 9 wherein the texture variations are in the form of hemispheres.

FIG.1

30

30

21

40

30

28

FIG. 2

22

21

15

21

40

28

20

25

3    3

FIG. 3

FIG. 4

FIG. 5

FIG. 6